Europäisches Patentamt

European Patent Office

Office européen des brevets

(11) Publication number: 0 303 233
A2

# (12) EUROPEAN PATENT APPLICATION

(21) Application number: 88112942.3

(22) Date of filing: 09.08.88

(51) Int. Cl.⁴: C12P 21/02 , C12N 15/00 , C12N 1/18 , A61K 37/02 , //(C12N1/18,C12R1:865)

(30) Priority: 10.08.87 JP 200514/87

(43) Date of publication of application: 15.02.89 Bulletin 89/07

(84) Designated Contracting States: AT BE CH DE ES FR GB GR IT LI LU NL SE

(71) Applicant: SHIONOGI & CO., LTD. 12, Dosho-machi 3-chome Higashi-ku Osaka 541(JP)

(72) Inventor: Okamoto, Hiroshi, Dr. med. 3-205, 2-15, Tsunogoro Sendai-shi Miyagi(JP)
Inventor: Itoh, Takako 4-45, Fujigaoka Takarazuka-shi Hyogo(JP)
Inventor: Teraoka, Hiroshi 2-47-10, Takakuradai Sakai-shi Osaka(JP)
Inventor: Tsuzuki, Hiroshige 5-12-15, Osumigaoka Tanabe-cho Tsuzuki-gun Kyoto(JP)
Inventor: Yoshida, Nobuo 5-8, Koshiensanban-cho Nishinomiya-shi Hyogo(JP)

(74) Representative: Vossius & Partner Siebertstrasse 4 P.O. Box 86 07 67 D-8000 München 86(DE)

(54) Human reg protein.

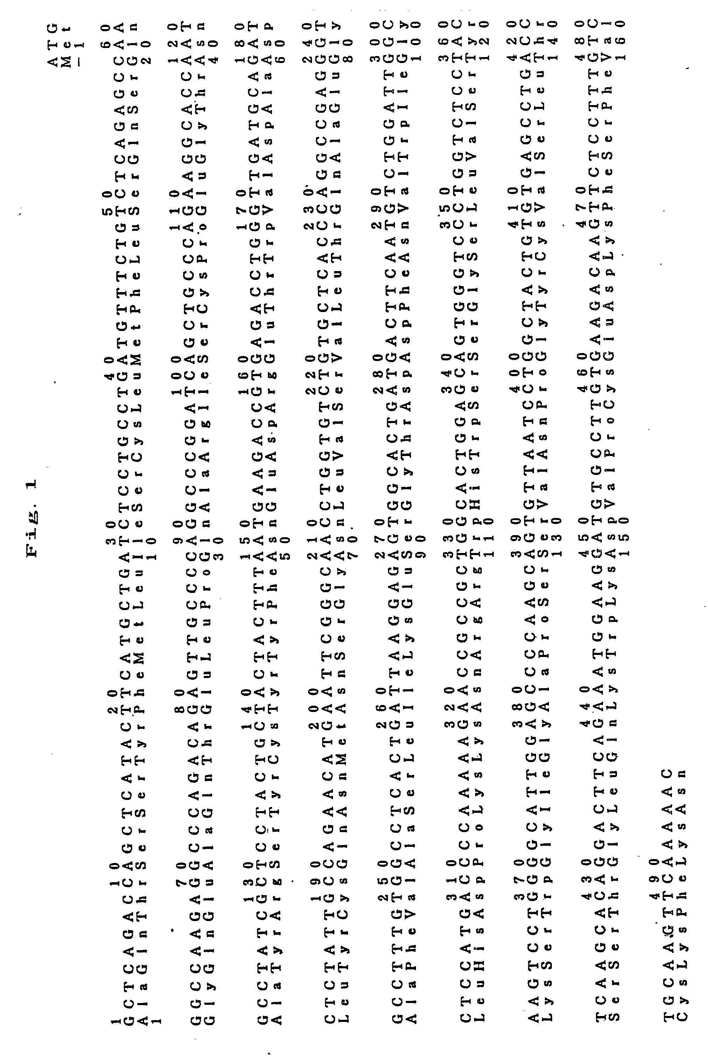

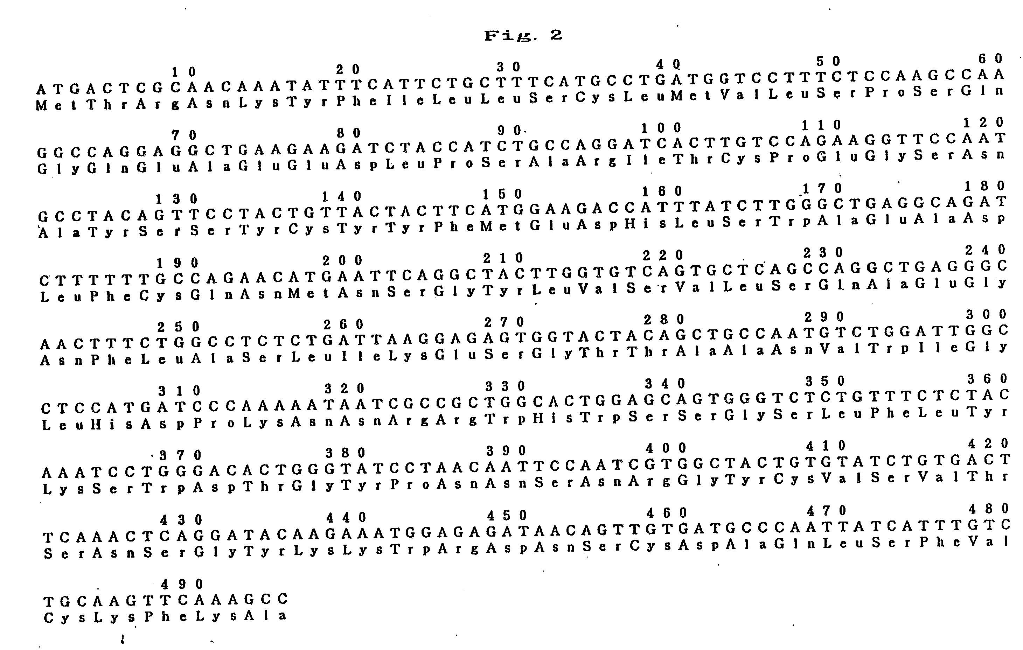

(57) A human reg protein is disclosed which comprises an amino acid sequence from Ala (1st), Gly (21st), Gln (22nd), Glu (23rd), Ala (24th), Gln (25th), Gln (30th), Ala (31st) or Ile (33rd) to Asn (165th) of the amino acid sequence shown in Fig. 1. Furthermore, a method for the production of said human reg proteins is disclosed.

EP 0 303 233 A2

Fig. 1

```
                                                                          A T G
                                                                          M e t
                                                                          - 1
        10            20            30            40            50            60
GCTCAGACCAGCTCATACTTCATGCTGATCTCCTGCCTGATGTTTCTGTCTCAGAGCCAA
AlaGlnThrSerSerTyrPheMetLeuIleSerCysLeuMetPheLeuSerGlnSerGln
1                                   10                            20

        70            80            90           100           110           120
GGCCAAGAGGCCCAGACAGAGTTGCCCCAGGCCCGGATCAGCTGCCCAGAAGGCACCAAT
GlyGlnGluAlaGlnThrGluLeuProGlnAlaArgIleSerCysProGluGlyThrAsn
                                        30                            40

       130           140           150           160           170           180
GCCTATCGCTCCTACTGCTACTACTTTAATGAAGACCGTGAGACCTGGGTTGATGCAGAT
AlaTyrArgSerTyrCysTyrTyrPheAsnGluAspArgGluThrTrpValAspAlaAsp
                                    50                            60

       190           200           210           220           230           240
CTCTATTGCCAGAACATGAATTCGGGCAACCTGGTGTCTGTGCTCACCCAGGCCGAGGGT
LeuTyrCysGlnAsnMetAsnSerGlyAsnLeuValSerValLeuThrGlnAlaGluGly
                                    70                            80

       250           260           270           280           290           300
GCCTTTGTGGCCTCACTGATTAAGGAGAGTGGCACTGATGACTTCAATGTCTGGATTGGC
AlaPheValAlaSerLeuIleLysGluSerGlyThrAspAspPheAsnValTrpIleGly
                                    90                           100

       310           320           330           340           350           360
CTCCATGACCCCAAAAAGAACCGCCGCTGGCACTGGAGCAGTGGGTCCCTGGTCTCCTAC
LeuHisAspProLysLysAsnArgArgTrpHisTrpSerSerGlySerLeuValSerTyr
                                   110                           120

       370           380           390           400           410           420
AAGTCCTGGGGCATTGGAGCCCCAAGCAGTGTTAATCCTGGCTACTGTGTGAGCCTGACC
LysSerTrpGlyIleGlyAlaProSerSerValAsnProGlyTyrCysValSerLeuThr
                                   130                           140

       430           440           450           460           470           480
TCAAGCACAGGACTTCAGAAAATGGAAGGATGTGCCTTGTGAAGACAAGTTCTCCTTTGTC
SerSerThrGlyLeuGlnLysTrpLysAspValProCysGluAspLysPheSerPheVal
                                   150                           160

       490
TGCAAGTTCAAAAAC
CysLysPheLysAsn
```

# Human Reg Protein

This invention relates to a human reg protein comprising an amino acid sequence from Ala (1st), Gly (21st), Gln (22nd), Glu (23rd), Ala (24th), Gln (25th), Gln (30th), Ala (31st) or Ile (33rd) to Asn (165th) among the amino acid sequence shown in Fig. 1 or an amino acid sequence further having Met at the N-terminus of said amino acid sequence and its production.

Insulin-producing pancreatic islet B cells have an extremely limited ability to regenerate. This creates a predisposition for the development of diabetes. In 1984, Okamoto and his colleagues successfully induced a regeneration of pancreatic islet B cells for the first time by daily injection of poly(ADP-ribose) synthetase inhibitors such as nicotinamide to 90% depancreatized rats (Yonemura, Y. et al., Diabetes 33, 401, 1984). Additionally, it has recently been reported that the remission phase of human insulin-dependent diabetes can be prolonged by oral administration of nicotinamide in large doses (Ph. Vague et al., Lancet Vol. I, No. 8533, 619-620, 1987).

The differential hybridization method as used in the present invention has already been established by Takasawa et al. (Diabetes 35, 1178, 1986). In this method, a gene expressed specifically in pancreatic B-cell tumors was successfully identified by differential screening of a rat B-cell tumor cDNA library using CDNA prepared from poly(A)$^+$ RNA of B-cell tumors and cDNA from poly(A)$^+$ RNA of normal pancreatic islets as probes.

In the priority interval we reported on the isolation of a gene specifically expressed in rat. The expression product regenerated pancreatic islet B cells. Furthermore, it was found that a human pancreas-derived cDNA library contains a homologue to the gene. This gene was named "reg" (regenerating gene) (J. Biol. Chem. 263, 2111-2114, 1988).

Administration of insulin is the only therapy for diabetes currently recognized. Oral hypoglycemic agents such as sulfonylurea, which were once expected to be effective, have been found to exhibit adverse side effects, including disturbance of the ability of pancreatic B cells to produce insulin and the induction of coronary arteriosclerosis with long-term administration. Thus, there is a need to develop an agent that regenerates human pancreatic islet B cells.

The technical problem underlying the present invention, therefore, is to provide polypeptides having human reg protein activity and processes for their production. A further object is to provide such proteins to be used as pharmaceuticals in particular for the regeneration of human pancreatic islet B cells.

These problems are solved by the present invention. Thus, the present invention relates to the subject matter of the claims and the following description.

According to the present invention, the rat reg and human reg, i.e. mammamlian regs can be produced in a microorganism by utilizing genetic engineering techniques. Thus, this invention provides human reg proteins encoded by the human reg. The human reg proteins of this invention seem to play an important role in the regeneration of insulin-producing pancreatic B cells. By mass-production of the proteins encoded by the human reg and developing corresponding useful pharmaceutical agents, it will be possible to open a new dimension in the treatment of diabetes, surpassing the conventional insulin administration therapy.

The Figures show:

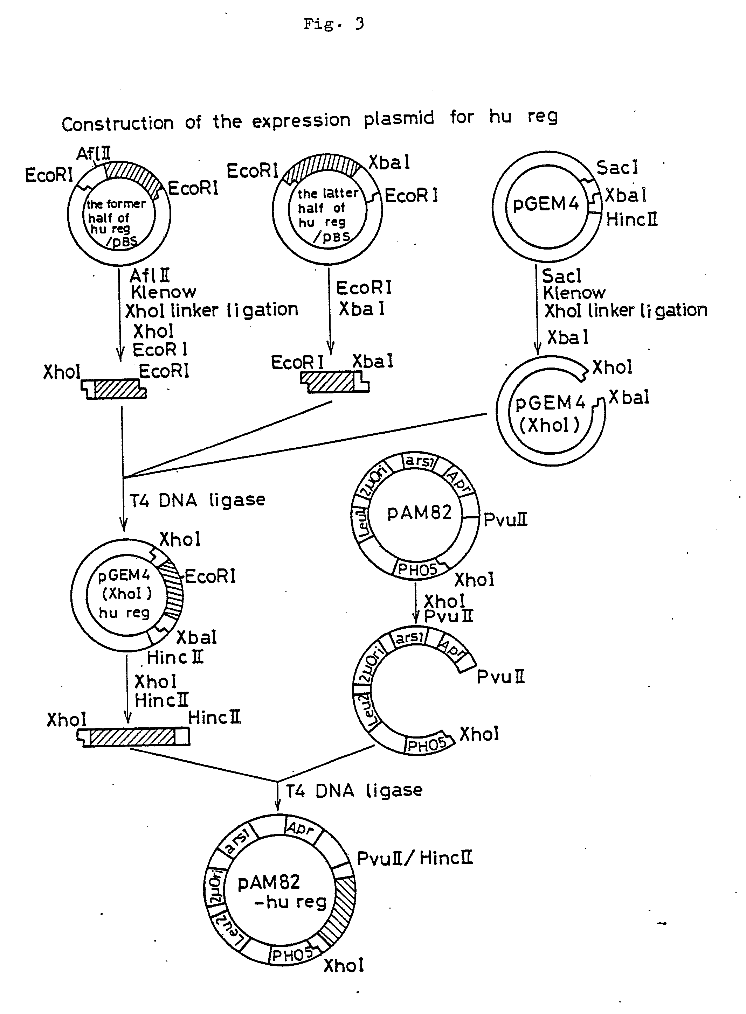

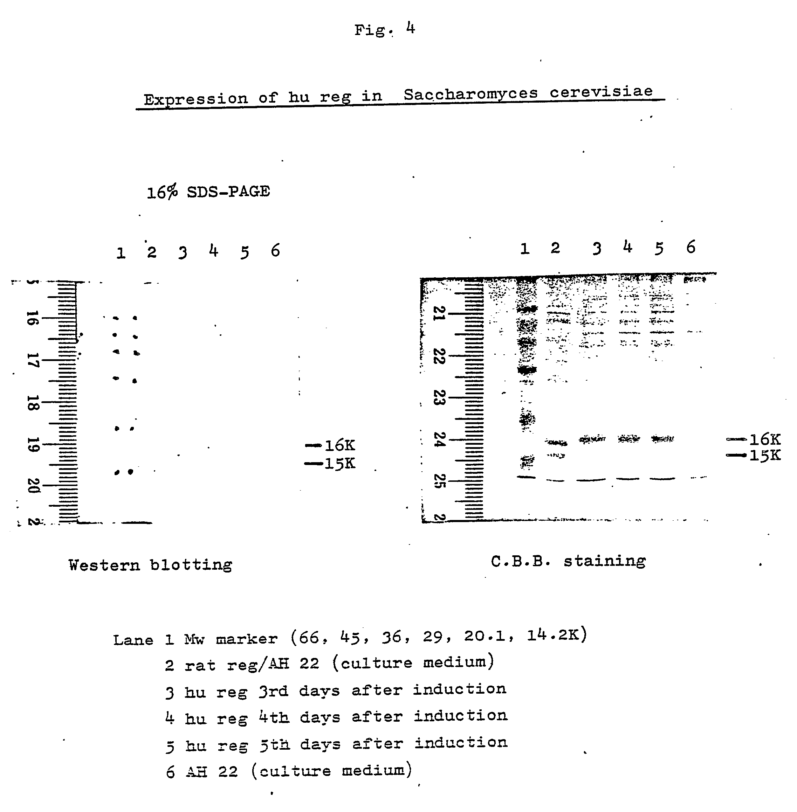

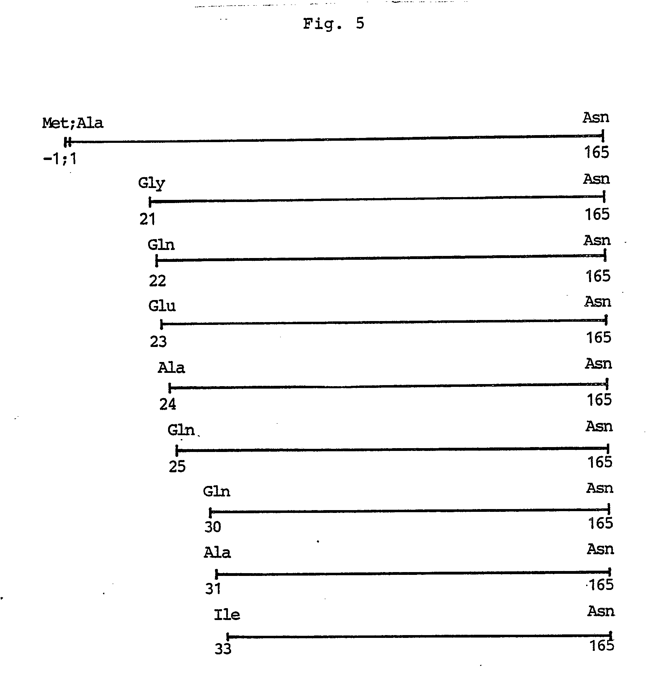

Fig. 1 shows the base sequence of the human reg cDNA and the amino acid sequence deduced therefrom. Fig. 2 shows the base sequence of the rat reg cDNA and the amino acid sequence deduced therefrom. Fig. 3 shows a construction of an expression plasmid for human reg proteins which is abbreviated as hu reg therein. Fig. 4 shows the results of SDS-PAGE of a 3-5 days-culture of yeast carrying the expression plasmid for human reg proteins.Fig. 5 shows preferred embodiments of the reg proteins of the present invention. The amino acid positions indicated correspond to those of Fig. 1.

In order to be able to study the mechanism of the regeneration and proliferation of pancreatic islet B cells, it was necessary to detect and isolate a gene which is specifically expressed in rats and which is responsible for regenerating pancreatic islet B cells. According to the present invention such a gene, named "reg", regenerating gene, was discovered. Furthermore, it was necessary to detect and isolate the corresponding human gene homologuous to the rat reg gene by using the rat reg gene as a probe from a human pancreatic cDNA library. Finally, it was necessary to develop processes for the expression of the human reg gene in a suitable host and recovering polypeptides having human reg protein activity. In an alternative embodiment, the regs of the present invention can be obtained by conventional DNA synthesis.

The rat reg used as a probe was prepared in the following manner.

I. Isolation of rat regenerating pancreatic islets

(1) 90% pancreatectomy

Male Wistar rats weighing 200 to 300 g were laparotomized under anesthesia with ether and the pancreas except for the parabiliary segment (corresponding to 90% of the whole pancreas) was resected, and the incised part was closed. The 90% pancreatectomy was established by V. G. Foglia (Rev. Soc. Argent. Biol. 20, 21-37, 1944) and it is widely used for the production of models of insulin-dependent diabetes. The rats fall into a diabetic state within a month after being subjected to 90% pancreatectomy.

(2) Administration of nicotinamide

Nicotinamide (50 mg/ml physiological saline) was intraperitoneally administered once a day at a dose of 0.5 g/kg body weight from 7 days before to 3 months after the 90% pancreatectomy. The administration of nicotinamide prevented and ameliorated the diabetic status of the 90% depancreatized rats.

(3) Isolation of regenerating islets

By the above steps (1) and (2), the regeneration of islets was induced in the remaining pancreas and both the number of islets per unit volume of pancreas and the size per islet increased, the increase in the number of islet cells being responsible for that in their size. Most of the increasing cells were insulin-producing B cells and the numbers of glucagon-producing A cells and somatostatin-producing D cells were not changed (Yonemura, Y. et al. Diabetes 33, 401, 1984). Three months after the operation the 90% depancreatized and nicotinamide-administered rats were laparotomized under anesthesia with Nembutal and the remaining pancreas was extirpated after being swollen with Hanks' solution. The extracted pancreata were cut into pieces in 5 ml of Hanks' solution per two remaining pancreata, 150 mg of bovine serum albumin (Sigma, Type V) and 40 mg of collagenase (Wako Pure Chemical Industries, Ltd., Type IV) were added, and the mixture was shaken for 3 to 5 minutes at 37°C for digestion. After the digestion, the solution was suspended in 50 ml of Hanks' solution and allowed to stand for 5 minutes for the islets to be precipitated. After the upper half of the Hanks' solution was removed and a further 25 ml of fresh Hanks' solution was added, the mixture was suspended again. After standing, removal and resuspension had been performed 8 times, the regenerating islets were collected under a stereoscopic microscope. Then, 1355 regenerating islets were collected from 32 rats which had been 90%-depancreatized and nicotinamide-treated. The above method basically follows the method reported by Okamoto (H. Okamoto, Molecular and Cellular Biochemistry 37, 43-61, 1981).

II. Preparation of a rat regenerating pancreatic islet complementary DNA (cDNA) library

(1) Isolation of RNA

Isolated regenerating islets were disrupted by sonication in 4M guanidinium thiocyanate, 10 mM sodium citrate (pH 7.0), 0.5% sodium lauryl sarcosine, 0.1 M 2-mercaptoethanol and 1% Antifoam A (Sigma). The mixture was layered onto cesium trifluoroacetic acid with a density of 1.64 (Pharmacia) and centrifuged for 14 to 16 hours at 25°C at 44,000 rpm. After the centrifugation, the RNA precipitate was recovered. From 1355 regenerating islets, 885 μg of RNA were obtained (Chirgwin, J. M. et al., Biochemistry 1979, 18: 5294-99).

(2) Isolation of poly(A)$^+$ RNA

From the RNA obtained in the above step (1), 17.2 μg of poly(A)$^+$ RNA were isolated by oligo(dT) cellulose column chromatography (H. Aviv & P. Leder, Proc. Natl. Acad. Sci. 69, 1408-1412, 1972).

## (3) Preparation of cDNA library

At first, by using 2 μg of poly(A)[+] RNA from regenerating islets as a template and oligo(dT) as a primer and by incubating them with 40 units of reverse transcriptase for an hour at 42°C, 182 ng of cDNA (first strand) were synthesized. The obtained cDNA-RNA hybrid was treated with 0.75 units of RNaseH, 46 units of DNA polymerase I and 4 units of T4 DNA polymerase to obtain 284 ng of double-stranded cDNA. The synthesis of cDNA stated above was carried out according to the method of U. Gubler et al. (Gene 25, 263-269, 1983).

cDNA (double-stranded) was treated with 15 units of EcoRI methylase (New England Biolabs) and ligated to EcoRI linker (450 ng) with 175 units of T4 DNA ligase (Takara Shuzo) for 45 hours at 13°C.

The above product was digested with 76 units of EcoRI (Takara Shuzo) for 2 hours at 37°C and applied onto Sephacryl S-1000 (Pharmacia) to be separated into cDNA and digested linker. By the above steps, 214 ng of EcoRI methylase-treated EcoRI fragment (double-stranded cDNA) were recovered.

20 ng of the above cDNA were litigated with 1.23 μg of λgt10 arms (dephosphorylated at 5' terminal by alkaline phosphatase after being digested by EcoRI, Stratagene) with the use of T4 DNA ligase (Takara Shuzo, 116 units) for 15 hours at 13°C. The ligated λgt10-regenerating islet cDNA was packaged with the use of Gigapack (Stratagene) and introduced into E. coli K12-derived Y1089 (DNA cloning, vol. 1, 49-78, 1985, IRL Press) and thus a total of 2.8 x 10[6] transformants were obtained.

III. Differential screening of the regenerating islet cDNA library:

Isolation of genes specifically expressed in regenerating islets

From each of 5000 independent plaques arbitrarily selected from regenerating islet cDNA libraries prepared in the above step II, phages were inoculated with toothpicks into NZY-0.2% maltose medium (1% NZ amine, 0.5% Bacto-yeast extract, 0.5% sodium chloride and 0.2% maltose) containing Y1089 corresponding to 0.5 OD$_{600}$ in microtiter plates (96 wells) and incubated overnight at 37°C for proliferation. Two μl of each phage solution obtained were spotted onto nitrocellulose filters (Schleicher and Schüll, BA85) and hybridized to rat insulin II cDNA labeled with $^{32}$P by nick translation. As a result, 16% of the clones were identified as insulin cDNA clones and the remaining 84% non-insulin cDNA clones were available for the subsequent screening.

Poly(A)[+] RNA (150 ng) from rat regenerating islets or from untreated rat normal islets were incubated for 2 hours at 37°C with 15 units of reverse transcriptase (Seikagaku Kogyo) in the presence of 41 ng of oligo(dT)$_{12-18}$, 20 mM dATP, 20 mM dTTP, 20 mM dGTP, 2 μM dCTP and 60 μCi[α-$^{32}$P]dCTP (Amersham). After the RNA chain was decomposed with 0.3N sodium hydroxide (at 100°C for 2 min.), the produced $^{32}$P- labeled single-stranded cDNA was recovered by means of ethanol precipitation. By this method, probes having a specific activity of 1 to 2 x 10[8] cpm/μg poly(A)[+] RNA were obtained.

Two sets of nitrocellulose filters were spotted with 2 μl samples of each phage solution of non-insulin cDNA clones, treated at room temperature with 0.5 N sodium hydroxide/1.5 M sodium chloride for 5 minutes and then with 0.5 M tris-hydrochloric acid (pH 8.0)/1.5 M sodium chloride for another 5 minutes, then dipped in 2 x SSC (1 x SSC: 0.15 M sodium chloride and 0.015 M sodium citrate) and baked for 2 hours at 80°C.

The baked nitrocellulose filters were allowed to react in 3 x SSC for 30 minutes at 65°C and then in 3 x SSC and 1 x FBP (Denhardt's solution; 0.02% Ficoll 400, 0.02% bovine serum albumin and 0.02% polyvinyl pyrrolidone) for an hour at 65°C and pre-hybridized at 65°C for an hour in 1 M sodium chloride, 50 mM tris-hydrochloric acid (pH 7.4), 10 mM EDTA, 0.1% SDS, 1 x FBP, 10 μg/ml salmon testis DNA and 250 μg/ml poly(U) (Pharmacia). Hybridization was carried out with the use of cDNA (1.5 x 10[7] cpm) prepared from rat regenerating islet poly(A)[+] RNA as a probe for one set of filters (stated before) and cDNA (1.5 x 10[7] cpm) prepared from normal islet poly(A)[+] RNA isolated from untreated rats as a probe (stated before) for the other set of filters in 1 M sodium chloride, 50 mM tris-hydrochloric acid (pH 7.4), 10 mM EDTA (pH 8.0), 0.1% SDS and 250 μg/ml poly(U) for 16 hours at 65°C.

After the hybridization, the filters were washed twice with 2 x SSC at room temperature for 1 to 2 minutes and further washed 4 times with 0.1 x SSC/0.1% SDS at 65% for 40 minutes and dried at 80°C for an hour and the autoradiography was carried out at -80°C overnight. Consequently, one kind of clone that consistently hybridized specifically to regenerating islet cDNA could be obtained.

When this invention is reproduced, this screening can easily be achieved by using a probe prepared by referring to the base sequence shown in Fig. 2. In an alternative embodiment the rat reg DNA sequence can be obtained by conventional DNA synthesis.

## IV. Sequencing of the gene specifically expressed in the regenerating islet B cells

Phage DNA was purified from the cDNA clone of the gene specifically expressed in the regenerating islets obtained in the above step III (see T.Maniatis et al., Molecular Cloning, Cold Spring Harbor Laboratory, New York (1982)), digested with EcoRI, and cDNA insert was isolated from phage vector arms by 1% agarose gel electrophoresis. The base sequence of the isolated cDNA was determined by Sanger's method using M13 (mp18 and mp19, Pharmacia) (Methods in Enzymology 101, 20-78, 1983).

As a result, it was found that the transcript expressed specifically in regenerating islets comprises a total length of 749 nucleotides, not including the poly(A) part, and encodes a protein composed of 165 amino acid residues starting with methionine and ending with alanine. The base sequence and deduced amino acid sequence are shown in Fig. 2.

Computer analysis of the nucleic acid and protein data bases (method of Wibur, W. J. & Lipman, D. J., Proc. Natl. Acad. Sci. USA 1983, 80, 726-730) of European Molecular Biology Laboratory (Heidelberg), GenBank (Cambridge, Massachusetts) and National Biomedical Research Foundation (Washington, DC) revealed that the base sequence and deduced amino acid sequence of the newly cloned cDNA of this invention were different from any known genes or gene products, and no gene or gene product showing 50% or more homology could be found in the data bases.

Accordingly, the cDNA cloned from the regenerating islet cDNA library was considered to correspond to a novel DNA fragment (gene) which has never been reported before and the gene was named "reg" (regenerating gene).

A human reg was obtained from a human pancreatic cDNA library in the following manner that utilizes a part of the above-mentioned rat reg as a probe.

## I. Isolation of human reg

## 1. Preparation of human pancreatic cDNA library

a) It is impossible to prepare human regenerating islets of Langerhans and therefore a cDNA library was constructed from human pancreatic tissue that had been obtained by operation. In rat pancreatic tissue is detected mRNA of reg.

### b) Isolation of RNA

Human pancreas obtained surgically was disrupted with Polytron in 4M guanidinium thiocyanate, 10 mM sodium citrate (pH 7.0), 0.5% sodium lauryl sarcosine, 0.1 M 2-mercaptoethanol and 1% Antifoam A (Sigma). The mixture was layered onto cesium trifluoroacetic acid (Pharmacia) with a density of 1.64 and centrifuged for 14 to 16 hours at 25° C at 44,000 rpm. After the centrifugation, the RNA precipitate was recovered. From 500 mg of pancreatic tissue, 2.526 mg of RNA was obtained (Chirgwin, J. M. et al., Biochemistry 1979, 18: 5294-99).

### c) Isolation of poly(A)$^+$ RNA

From the RNA obtained in the above step (b), 17.56 μg of poly(A)$^+$ RNAwere isolated by oligo(dT) cellulose column chromatography (H. Aviv. & P. Leder, Proc. Natl. Acad. Sci. 69, 1408-1412, 1972).

d) Preparation of cDNA library

At first, by using 1 μg of human pancreatic poly(A)⁺ RNA as a template and oligo(dT) as a primer and by incubating them with 40 units of reverse transcriptase for an hour at 42°C, about 100 ng of cDNA (first strand)were synthesized. The obtained cDNA-RNA hybrid was treated with 0.75 units of RNaseH, 46 units of DNA polymerase I and 4 units of T4 DNA polymerase to obtain about 150 ng of double-stranded cDNA. The synthesis of cDNA described above was carried out basically according to the method of U. Gubler et al. (Gene 25, 263-269, 1983).

cDNA (double-stranded) was treated with 15 units of EcoRI methylase (New England Biolabs) and ligated to EcoRI linker (450 ng) with 175 units of T4 DNA ligase (Takara Shuzo) for 45 hours at 13°C.

The above product was digested with 76 units of EcoRI (Takara Shuzo) for 2 hours at 37°C and applied onto Sephacryl S-1000 (Pharmacia) to be separated into cDNA and digested linker. By the above steps, 17.6 ng of EcoRI methylase-treated EcoRI fragment (double-stranded cDNA) were recovered.

The above cDNA was ligated to 1.0 μg of λgt10 arms (dephosphorylated at 5′ terminal by alkaline phosphatase after having been digested with EcoRI, Stratagene) with T4 DNA ligase (Takara Shuzo, 116 units) for 15 hours at 13°C. The ligated λgt10-human pancreatic cDNA was packaged with Gigapack (Stratagene), introduced into E. coli K12-derived Y1089 (DNA cloning, vol. 1, 49-78, 1985, IRL Press) and thus a total of 6 x 10⁵ transformants was obtained.

## 2. Cloning of human reg cDNA from human pancreatic cDNA library

a) From each plaque of the human pancreatic cDNA library prepared, phages were inoculated into maltose medium (0.2% maltose, 1% NZ amine, 0.5% Bacto-yeast extract and 0.5% sodium chloride) containing Y1089 corresponding to 0.5 OD$_{600}$ and incubated overnight at 37°C for proliferation. As a result, 10⁶ plaques were formed on an agar medium (150 mm in diameter) and transferred onto nitrocellulose filters.

b)An oligodeoxyribonucleotide (60 bases) corresponding to the base sequence from 76th to 135th of rat reg (Fig. 2) was synthesized with a DNA synthesizer (Applied Biosystems Model 380 B) and its 5′ terminal was labelled with ³²P by using [γ -³²P] ATP and T4 polynucleotide kinase.

c) Nitrocellulose filters obtained in the above step a) were treated at room temperature with 0.5 N sodium hydroxide/1.5 M sodium chloride for 5 minutes and then with 0.5 M tris-hydrochloric acid (pH 8.0)-/1.5 M sodium chloride for another 5 minutes, then dipped in 2 x SSC (1 x SSC: 0.15 M sodium chloride and 0.015 M sodium citrate) and baked for 2 hours at 80°C. The baked nitrocellulose filters were pre-hybridized at 50°C for 4 hours in 6 x SSC, 5 x FBP (1 x FBP; 0.02% Ficoll 400, 0,02% bovine serum albumin and 0.02% polyvinyl pyrrolidone), 0.1% SDS and 200 μg/ml E. coli tRNA, hybridized with the ³²P-labeled oligodeoxyribonucleotide at 50°C for 12-16 hours in 6 x SSC, 1 x FBP, 0.1% SDS and 100 μg/ml E. coli tRNA containing 2 ng/ml ³²P-labeled oligodeoxyribonucloetide and washed 4 times, 30 minutes each time, at 50°C with 6 x SSC and 0.1% SDS.

d) As a result, 58 clones among 10⁶ human pancreatic cDNA clones hybridized with a rat reg probe of 60 bases.

e) From the positive clones, the clone with the longest cDNA insert (about 900 nucleotides) was selected. The DNA insert was isolated from the clone, digested with EcoRI and then sub-cloned in the pBS vector (Stratagene)at the EcoRI site. Because human reg has an internal EcoRI site, it was divided into two fragments i.e. the 5′-and 3′- terminal halves by EcoRI digestion. These two fragments were subcloned into pBS vector, respectively. In an alternative embodiment the human reg DNA sequence can be obtained by conventional DNA synthesis.

## II. Sequencing of the human reg cDNA

a) The base sequence of the cDNA sub-cloned in the pBS vector was determined according to Sanger's method (Methods in Enzymology 101, 20-78, 1983).

b) As a result, it was found that the human reg cDNA comprises a total length of 749 nucleotides, not including the poly(A) parts, and encodes a protein composed of 166 amino acid residues starting with methionine and ending with asparagine.

c) The protein encoded by the human reg is longer by one amino acid residue than the rat reg-encoded protein composed of 165 amino acid residues. The base sequence of the coding region of the human reg and the amino acid sequence deduced therefrom show 75% and 68% homologies with those of the rat reg, respectively.

d) The sequence at the N-terminus of the human reg protein is rich in hydrophobic amino acid residues. This is the main characteristic of the signal peptides of secretory proteins. Consequently, in the human reg protein the sequence starting with Met (-1) and ending with either Gln (20), Gly (21), Gln (22), Glu (23), Ala (24), Pro (29), Gln (30), Arg (32) was deduced to be a signal peptide.

The obtained cDNA can be expressed by a common method. For example, the cDNA can be expressed when it is inserted in an appropriate expression vector (pKK223-3, pBS, pDR540, pDR720, pPL-lambda etc.)under the control of an appropriate promoter (lac, Tac, Trp, $P_L$ and others) and introduced into a host (E. coli K-12 AG-1, MM294, DH1, HB101, C600 etc.). When eukaryotic cells such as yeast and simian cells are used as a host, it is easier to recover the desired protein from the culture because it is possible to have the desired protein secreted into the medium. For example, when yeast is used, various known vectors such as pGPD-1, pMFα8, AAR6, ABade8, AH5, YEp52, pACF301 and pAM82 can be used as expression vectors. If simian COS cells are used, pKSV-10 and others are usable as expression vectors. Aside from the above host-vector systems, most of the known host-vector systems are suitable for the expression of reg.

The expressed reg protein may be purified according to conventional purifying methods by appropriately combining centrifugation, chromatography, dialysis, salting-out and other methods.

The reg protein encoded by the reg gene of this invention is not restricted to the protein that has the amino acid sequence shown in Fig. 1. There appears a signal sequence at the amino terminus of the amino acid sequence and the mature reg protein seems to begin with Gly (21st), Gln (22nd), Glu (23rd), Ala (24th), Gln (25th), Gln (30th), Ala (31st) or Ile (33rd). Therefore, such mature reg proteins are included in this invention. When a protein is manufactured by using gentic engineering techniques, methionine is often added to the N-terminus of the protein. Therefore, the above proteins which additionally contain a methionine residue at their N-terminus are also included in this invention.

Variants of the reg proteins having the amino acid sequence given in Fig. 1 or Fig. 2 can be obtained by expressing in a suitable host organism a corresponding DNA sequence which is obtainable as an allelic derivative of the DNA sequences shown in Fig. 1 or Fig. 2 from nature by conventional DNA synthesis or by in vitro mutation of the DNA sequences shown in said Figures.

Particularly preferred embodiments of the present invention are reg proteins which contain the amino acid positions 22 (Gln), 23 (Glu) or 33 (Ile) to 165 (Asn) of the amino acid sequence shown in Fig. 1.

## Example

Expression of "human reg" in Saccharomyces cerevisiae

## Materials and Methods

### Strains and Media

Escherichia coli K-12, strain C600 (F⁻, hsdR⁺, hsdM⁺ recA⁺, thr, leu, thi, lacY, supE, tonA) was used for host of plasmid constructions.

Saccharomyces cerevisiae, strain AH22 ( a leu2, his4, can1, cir⁺) (Proc. Natl. Acad. Sci. USA 75, 1929-1933 (1978)) was used as the recipient strain for a plasmid.

Yeast cells were grown at 30°C in YPDA medium (1% yeast extract, 2% polypeptone, 2% glucose and 20 mg/l Adenine).

Burkholder's minimal medium (Proc. Natl. Acad. Sci. USA 77, 4504-4508 (1980)) supplemented with histidine (20 mg/l) was used for preparation of Pi-enriched medium containing 1.5 g $KH_2PO_4$ per liter (abbreviated Pi($^+$) medium) or Pi-deficient medium containing 1.5 g KCl per liter in place of Pi (abbreviated Pi($^-$) medium).

## Enzymes and a Linker

Restriction enzymes, T4DNA ligase and Klenow Fragment were purchased from Takara Bio Chemicals (Kyoto, Japan). Xho I linker was purchased from Pharmacia (Tokyo, Japan).

## Plasmids

A yeast-E.coli shuttle vector pAM82 (Proc. Natl. Acad. Sci. USA 80, 1-5, (1983) EP-B 0 103 201, JP-B 61-55951) was used. The Saccharomyces cerevisiae AH22 carrying the vector pAM82 has been deposited under the Budapest Treaty with the Fermentation Research Institute at 1-3, Higashi 1 chome Tsukuba-shi Ibaraki-ken 305, Japan, as Saccharomyces cerevisiae AH22/pAM82 under the deposition number FERM BP-313 on August 16, 1982. It consists of a 5.5 kb fragment from S. cerevisiae DNA carrying ARS1, 2-μm ori and Leu 2 along with a 3.7 kb fragment from E.coli plasmid pBR322 carrying the $Ap^R$ marker and the ori. In addition it carries a 2.7 kb yeast DNA fragment containing the PHO5 promoter region.

Plasmid pGEM4 was purchased from Promega Biotec (U.S.A.). Human reg cDNA (J. Biol. Chem. 263, 2111-2114 (1988)) has an EcoRI site in the structural gene and two EcoRI fragments were therefore inserted into the EcoRI site of the pBS vector, respectively.

## Transformation of Yeast Cells

Transformation was performed as described by Kimura et al (J. Bacteriol. 153, 165-168 (1983)). The Leu$^+$ transformants were selected on Burkholder's minimal medium containing 2% agar.

## Yeast Cell Growth and Induction

The acid phosphatase promoter (PHO5) is shown to be controlled by Pi concentration in the medium (The EMBO Journal 1, 675-680 (1982)).

First of all, yeast cells were grown in Pi($^+$) Burkholder's minimal medium supplemented with histidine (20 μg/ml) at 30° C with aeration for 2 days, and then an aliquot (200 μl) was transferred in 10 ml of Pi($^-$) medium supplemented with histidine (20 μg/ml).

It took about 3 to 5 days before human reg protein could be detected in the culture medium.

## Construction of the Expression Plasmid

Standard DNA techniques were used (Maniatis et al., Molecular Cloning, Cold Spring Harbor Laboratory, New York (1982)). A human reg expression plasmid was constructed as shown in Fig 3. Human reg cDNA subcloned into the pBS vector consisted of two parts i.e., the 5′- and 3′- terminal halves (the former and latter halves), respectively.

First the 5′-terminal half was digested wth Afl II, treated with Klenow Fragment, Xho I linkers were attached and then it was digested with EcoRI. Next, the 3′-terminal half was digested with EcoRI and Xba I. The resulting two fragments of the human reg cDNA were inserted into pGEM4(Xho I) whose Sac I site was previously exchanged into an Xho I site by using an Xho I linker.

Then pGEM4(Xho I) human reg was digested with Xho I and Hinc II, inserted into the pAM82 (digested with Xho I and Pvu II) vector.

This plasmid was designated as pAM82-human reg.

Each restriction endonuclease was used at the concentration of 3U/ μ gDNA, and incubated at 37° C for 1 hr.

## Production of human reg proteins

The expression plasmid for human reg cDNA constructed as described above was transformed into E.coli K-12, strain C600, and ampicillin resistant colonies were selected. Using these transformants a large amount of plasmid DNA was prepared.

The recombinant plasmid propagated in E.coli cells was transformed into the yeast recipient S.cerevisiae strain AH22, by a standard transformation procedure followed by selecting for Leu$^+$ colonies.

Yeast cells carrying plasmids were grown in Pi($^+$) medium for two days and induced by transferring into Pi($^-$) medium.

In both Pi($^+$) and Pi($^-$) medium yeast cells were incubated at 30°C with vigorous shaking.

In the 3rd, 4th and 5th day after incubating in Pi($^-$) medium, aliquots of the broth were withdrawn and centrifuged to remove the cells. The obtained culture medium was concentrated to approximately 100-fold by Centricon-10 (Amicon) and N$_2$ gas bubbling.

The condensed samples (equivalent to 0.75 ml of culture medium) were analysed on 16% SDS-PAGE by staining with Coomassie Brilliant Blue R.

As shown in Fig. 4, the human reg protein was found to be produced and secreted from the cells into the culture medium. The predominant protein is the human reg protein, while a small amount of concomitant proteins are also secreted.

## Immunological detection of human reg proteins in culture medium

Yeast culture medium was concentrated to 100-fold by Centricon-10 (Amicon) and N$_2$ gas bubbling.

The condensed medium was electrophoresed by 16% SDS-polyacrylamide gel (SDS-PAGE) (FEBS Lett. 58, 254-258 (1975)) (Nature (London) 277, 680 (1970)) and then Western blot analysis (J. Virol. 10, 211 (1972)) was performed using antibodies which are specific for the human reg protein.

As shown in Fig. 4, two different bands having approximate 15K-Da and 16K-Da were visualized, by which the main proteins secreted from the yeast host were identified as reg proteins.

## Purification and Characterization of the Reg Protein

## Purification of reg protein from yeast culture medium

One liter of yeast culture medium containing the reg protein was adjusted to pH 3.4 by adding 1 N HCl, and diluted with distilled water to a final conductivity of 0.3 mmho. To this solution 100 ml of S-Sepharose (fast flow type) previously equilibrated with 5 mM sodium acetate buffer (pH 3.4) (Buffer A) were added, and the mixture was gently agitated at 4°C for 16 h. S-Sepharose, which adsorbed the reg protein, was packed to a column (5 x 8 cm) and washed with Buffer A containing 0.3 M NaCl, and the reg protein was eluted from the column with Buffer A containing 0.6 M NaCl. The reg protein fractions, which display two different molecular weights of about 15,000 and about 16,000 on SDS-polyacrylamide gel electrophoresis (SDS-PAGE) (15% gel), were pooled and dialyzed against Buffer A at 4°C for 16 hr. The dialyzed material was applied to a S-Sepharose column (0.7 x 25 cm) equilibrated with Buffer A, and the column was washed with Buffer A containing 0.3 M NaCl. The reg protein was eluted with a linear gradient of 10 times column volume of Buffer A containing 0.3 M NaCl to Buffer A containing 0.6 M NaCl. Reg proteins of 15K and 16K were eluted with Buffer A containing 0.47 M and 0.44 M NaCl concentrations, respectively, each of which was homogeneous on SDS-PAGE (15% gel).

The purified reg proteins were concentrated and desalted by ultrafiltration with Amicon YM-10 membrane, and stored in 10 mM AcOH with concentration of 0.1 mg/ml. About 200 μg of reg protein were obtained with 1 liter of yeast culture medium.

## Characterization of the purified reg proteins

9

## (a) Molecular weight

The molecular weights of purified reg proteins of 15K and 16K were determined by SDS-PAGE to be about 14,500 and about 16,000, respectively, which are in line with the theoretical molecular weights of 15,023 and 16,275 caluculated from the amino acid sequence deduced from the cDNA (N-termini of 15K-reg protein and 16K-reg protein were inferred to be Ile at the 33rd position and Gln at the 22nd in Fig. 1, respectively.).

## (b) Amino acid composition

A portion of the 16K-reg protein was desalted with HPLC (Brownlee, aquapore RP-300 column) and hydrolyzed with 4 M methanesulfonic acid (containing 0.2% 3-(2-aminoethyl)indole) at 110°C for 24 h. Amino acid analysis was performed with a Hitachi amino acid analyzer (model 835).

As shown in Table I, the amino acid composition of the 16K-reg protein almost matched the theoretical values.

## (c) Partial N-terminal amino acid sequence of reg protein

The amino acid sequence of 16K-reg protein was attempted to be determined with an Applied Biosystems 477A Protein Sequencer. However, N-terminal and the following amino acids were not detected. Therefore, after being treated with pyroglutamate aminopeptidase, the 16K-reg protein was sequenced in the above manner. As shown in Table II, the N-terminal 29 amino acid residues of the enzyme-treated protein were determined. The N-terminal amino acid of the protein was glutamic acid, and the amino-terminal sequence (Glu-2 to Glu-30) corresponded to that deduced from the cDNA.

Therefore, the N-terminal amino acid of 16K-reg protein is

Gln (22nd) or Glu (23rd)

(Note: The N-terminal amino acid of one of the rat reg proteins is Glu corresponding to the Glu (23rd) of the human reg protein). With respect to the 15K-reg protein, it was found that after the expressed 16K-reg protein is exposed to a trypsin-like enzyme, it is converted to the 15K-reg protein. Therefore, the N-terminal amino acid of the 15K-reg protein is Ile (33rd) after Arg (32nd).

Table I

| Amino acid | Observed | Theoretical |
|------------|----------|-------------|
| Asp | 17.1 | 17 |
| Thr | 6.9 | 7 |
| Ser | 15.7 | 17 |
| Glu | 14.8 | 14 |
| Pro | 6.4 | 6 |
| Gly | 10.2 | 10 |
| Ala | 8.0 | 8 |
| 1/2Cys | 5.5 | 6 |
| Val | 9.9 | 10 |
| Met | 1.0 | 1 |
| Ile | 3.9 | 4 |
| Leu | 8.1 | 8 |
| Tyr | 7.1 | 7 |
| Phe | 7.0 | 7 |
| Lys | 9.1 | 9 |
| His | 2.2 | 2 |
| Arg | 4.8 | 5 |
| Trp | 5.8 | 6 |
| Total | | 144 |

Table II

| Degradation step | Reg protein residue | Recovery (%) |
|---|---|---|
| 1 | (Gln)*1 | |
| 2 | Glu | 17.3 |
| 3 | Ala | 18.1 |
| 4 | Gln | 20.6 |
| 5 | Thr | 11.9 |
| 6 | Glu | 14.9 |
| 7 | Leu | 16.8 |
| 8 | Pro | 15.2 |
| 9 | Gln | 14.7 |
| 10 | Ala | 14.2 |
| 11 | Arg | 7.9 |
| 12 | Ile | 9.8 |
| 13 | Ser | 35.6 |
| 14 | (Cys)*2 | |
| 15 | Pro | 7.5 |
| 16 | Glu | 5.7 |
| 17 | Gly | 8.6 |
| 18 | Thr | 12.2 |
| 19 | Asn | 5.9 |
| 20 | Ala | 6.8 |
| 21 | Tyr | 5.6 |
| 22 | Arg | 3.5 |
| 23 | Ser | 22.0 |
| 24 | Tyr | 4.5 |
| 25 | (Cys)*2 | |
| 26 | Tyr | 4.8 |
| 27 | Tyr | 6.4 |
| 28 | Phe | 4.4 |
| 29 | Asn | 4.1 |
| 30 | Glu | 2.7 |

*1 unidentified residue (Gln deduced from pyroglutamate aminopeptidase experiment)

*2 unidentified residue (Cys deduced from cDNA)

**Claims**

1. A reg protein selected from the following group of

a) a protein comprising the amino acid sequence from amino acid position 1 (Ala) to 165 (Asn) as given in Fig. 1;

b) a protein comprising the amino acid sequence from amino acid position 21 (Gly) to 165 (Asn) as given in Fig. 1;

c) a protein comprising the amino acid sequence from amino acid position 22 (Gln) to 165 (Asn) as given in Fig. 1;

d) a protein comprising the amino acid sequence from amino acid position 23 (Glu) to 165 (Asn) as given in Fig. 1;

e) a protein comprising the amino acid sequence from amino acid position 24 (Ala) to 165 (Asn) as given in Fig. 1;

f) a protein comprising the amino acid sequence from amino acid position 25 (Gln) to 165 (Asn) as given in Fig. 1;

g) a protein comprising the amino acid sequence from amino acid position 30 (Gln) to 165 (Asn) as given in Fig. 1;

h) a protein comprising the amino acid sequence from amino acid position 31 (Ala) to 165 (Asn) as given in Fig. 1;

i) a protein comprising the amino acid sequence from amino acid position 33 (Ile) to 165 (Asn) as given in Fig. 1;

j) a protein according to a) to i) which additionally comprises a Met-residue at its N-terminus;

k) a protein which in respect to a) to j) is deficient in one or more amino acid(s);

l) a protein in which in respect to a) to j) one or more amino acid(s) are replaced;

m) a fusion protein comprising a protein according to a) to l) in which the additional amino acids do not interfere with the biological reg-activity or may easily be eliminated; and

n) a protein which is an allelic derivative of a protein according to a) to j).

2. A DNA sequence encoding a reg protein according to claim 1.

3. The DNA sequence according to claim 2 which is the DNA sequence given in Fig. 1.

4. A recombinant DNA molecule, preferably an expression vector containing a DNA sequence according to claim 2 or 3.

5. A host cell being transformed with a recombinant DNA molecule according to claim 4.

6. The host cell according to claim 5, which is Saccharomyces cerevisiae.

7. A method for preparing the protein of claim 1 or 2 which comprises cultivating a host cell according to claim 5 or 6 and recovering said protein from the culture.

8. A pharmaceutical composition comprising a reg protein according to claim 1.

9. A pharmaceutical composition for the regeneration of pancreatic islet B cells comprising a reg protein according to claim 1.

## Fig. 1

```
                                                                              A T G
                                                                              M e t
                                                                              - 1

          10            20            30            40            50            60
1 GCTCAGACCAGCTCATACTTCATGCTGATCTCCTGCCTGATGTTTCTGTCTCAGAGCCAA
  AlaGlnThrSerSerTyrPheMetLeuIleSerCysLeuMetPheLeuSerGlnSerGln
1                                   10                                         20

          70            80            90           100           110           120
  GGCCAAGAGGCCCAGACAGAGTTGCCCCAGGCCCGGATCAGCTGCCCAGAAGGCACCAAT
  GlyGlnGluAlaGlnThrGluLeuProGlnAlaArgIleSerCysProGluGlyThrAsn
                                    30                                         40

         130           140           150           160           170           180
  GCCTATCGCTCCTACTGCTACTACTTTAATGAAGACCGTGAGACCTGGGTTGATGCAGAT
  AlaTyrArgSerTyrCysTyrTyrPheAsnGluAspArgGluThrTrpValAspAlaAsp
                                    50                                         60

         190           200           210           220           230           240
  CTCTATTGCCAGAACATGAATTCGGGCAACCTGGTGTCTGTGCTCACCCAGGCCGAGGGT
  LeuTyrCysGlnAsnMetAsnSerGlyAsnLeuValSerValLeuThrGlnAlaGluGly
                                    70                                         80

         250           260           270           280           290           300
  GCCTTTGTGGCCTCACTGATTAAGGAGAGTGGCACTGATGACTTCAATGTCTGGATTGGC
  AlaPheValAlaSerLeuIleLysGluSerGlyThrAspAspPheAsnValTrpIleGly
                                    90                                        100

         310           320           330           340           350           360
  CTCCATGACCCCAAAAAGAACCGCCGCTGGCACTGGAGCAGTGGGTCCCTGGTCTCCTAC
  LeuHisAspProLysLysAsnArgArgTrpHisTrpSerSerGlySerLeuValSerTyr
                                   110                                        120

         370           380           390           400           410           420
  AAGTCCTGGGGGCATTGGAGCCCCAAGCAGTGTTAATCCTGGCTACTGTGTGAGCCTGACC
  LysSerTrpGlyIleGlyAlaProSerSerValAsnProGlyTyrCysValSerLeuThr
                                   130                                        140

         430           440           450           460           470           480
  TCAAGCACAGGACTTCAGAAATGGAAGGATGTGCCTTGTGAAGACAAGTTCTCCTTTGTC
  SerSerThrGlyLeuGlnLysTrpLysAspValProCysGluAspLysPheSerPheVal
                                   150                                        160

         490
  TGCAAGTTCAAAAAC
  CysLysPheLysAsn
```

Fig. 2

EP 0 303 233 A2

```
                10                  20                  30                  40                  50                  60
ATGACTCGCAACAAATATTTCATTCTGCTTTCATGCCTGATGGTCCTTTCTCCAAGCCAA
MetThrArgAsnLysTyrPheIleLeuLeuSerCysLeuMetValLeuSerProSerGln

                70                  80                  90                 100                 110                 120
GGCCAGGAGGCTGAAGAAGATCTACCATCTGCCAGGATCACTTGTCCAGAAGGTTCCAAT
GlyGlnGluAlaGluGluAspLeuProSerAlaArgIleThrCysProGluGlySerAsn

               130                 140                 150                 160                 170                 180
GCCTACAGTTCCTACTGTTACTACTTCATGGAAGACCATTTATCTTGGGCTGAGGCAGAT
AlaTyrSerSerTyrCysTyrTyrPheMetGluAspHisLeuSerTrpAlaGluAlaAsp

               190                 200                 210                 220                 230                 240
CTTTTTTGCCAGAACATGAATTCAGGCTACTTGGTGTCAGTGCTCAGCCAGGCTGAGGGC
LeuPheCysGlnAsnMetAsnSerGlyTyrLeuValSerValLeuSerGlnAlaGluGly

               250                 260                 270                 280                 290                 300
AACTTTCTGGCCTCTCTGATTAAGGAGAGTGGTACTACAGCTGCCAATGTCTGGATTGGC
AsnPheLeuAlaSerLeuIleLysGluSerGlyThrThrAlaAlaAsnValTrpIleGly

               310                 320                 330                 340                 350                 360
CTCCATGATCCCAAAAATAATCGCCGCTGGCACTGGAGCAGTGGGTCTCTGTTTCTCTAC
LeuHisAspProLysAsnAsnArgArgTrpHisTrpSerSerGlySerLeuPheLeuTyr

               370                 380                 390                 400                 410                 420
AAATCCTGGGACACTGGGTATCCTAACAATTCCAATCGTGGCTACTGTGTATCTGTGACT
LysSerTrpAspThrGlyTyrProAsnAsnSerAsnArgGlyTyrCysValSerValThr

               430                 440                 450                 460                 470                 480
TCAAACTCAGGATACAAGAAATGGAGAGATAACAGTTGTGATGCCCAATTATCATTTGTC
SerAsnSerGlyTyrLysLysTrpArgAspAsnSerCysAspAlaGlnLeuSerPheVal

               490
TGCAAGTTCAAAGCC
CysLysPheLysAla
```

Fig. 3

Construction of the expression plasmid for hu reg

Fig. 4

Expression of hu reg in  Saccharomyces cerevisiae

16% SDS-PAGE

Western blotting

C.B.B. staining

Lane 1 Mw marker (66, 45, 36, 29, 20.1, 14.2K)
2 rat reg/AH 22 (culture medium)
3 hu reg 3rd days after induction
4 hu reg 4th days after induction
5 hu reg 5th days after induction
6 AH 22 (culture medium)

Fig. 5